# EUROPEAN PATENT APPLICATION

(11) **EP 4 589 602 A1**
(43) Date of publication of application: **23.07.2025**
(21) Application number: 24305099.4
(22) Date of filing: 16.01.2024
(51) Int. Cl.: G16H 50/20, G16H 50/30

(54) **PREDICTING HEART DISEASE**

(71) Applicant: Koninklijke Philips N.V., 5656 AG Eindhoven (NL)
(72) Inventor: CARBONATI, Tanner, 5656 Eindhoven (NL); ESLAMI, Parastou, 5656 Eindhoven (NL); PORQUET, Pauline, 5656 Eindhoven (NL); FIORINA, Laurent, 5656 Eindhoven (NL); HENRY, Christine, 5656 Eindhoven (NL); CHAUDHARI, Ashish, 5656 Eindhoven (NL); PEZZOTTI, Nicola, 5656 Eindhoven (NL)
(74) Representative: Philips Intellectual Property & Standards

(57) **Abstract**

Proposed concepts thus aim to provide schemes, solutions, concepts, designs, methods and systems pertaining to predicting a probability of heart disease in a subject. In particular, embodiments aim to provide a method for predicting a probability of heart disease in a subject by providing an ECG signal of a subject to a trained machine-learning model which can predict and output a probability of the subject having at least one RWMA and/or global RV hypokinesis.

## Description

### FIELD OF THE INVENTION

This invention relates to the field of predicting heart disease in a subject.

### BACKGROUND OF THE INVENTION

Ischemic heart disease (IHD) remains the leading cause of death and disability worldwide. IHD causes a wide spectrum of symptoms, and some patients with undiagnosed coronary artery disease may present without symptoms or access to diagnostic electrocardiography, which may delay diagnosis and intervention. Once identified, interventions such as medications and revascularization can be initiated to improve patient outcomes. Echocardiography is an important non-invasive diagnostic tool for identifying patients with IHD through its ability to identify global ventricular dysfunction and regional wall motion abnormalities (RWMAs) that correlate with prior myocardial infarction or areas with hibernating myocardium due to inadequate blood flow. Assessment of RWMA can help in the diagnosis of acute and chronic myocardial infarction, as well as differentiating between ischemic and non-ischemic cardiomyopathy.

Echocardiography is the most common diagnostic modality to determine RWMAs, however, it is unclear when initial or repeated imaging should be performed, as ischemic disease progresses at different rates. Furthermore, access to echocardiography may be limited in some clinical settings due to lack of resources or expertise. The electrocardiogram (ECG), however, is a widely available and inexpensive diagnostic tool that is commonly used to evaluate patients with suspected acute coronary syndrome in need of echocardiography or coronary angiography.

### SUMMARY OF THE INVENTION

The invention is defined by the claims.

According to examples in accordance with an aspect of the invention, there is provided a method for predicting a probability of heart disease in a subject.

The method comprises: providing an ECG signal of the subject as an input to a trained machine-learning model, the trained machine-learning model being trained to predict, based on the ECG signal, a heart disease risk score indicating a probability of the subject having at least one regional wall motion abnormality (RWMA) and/or a probability of the subject having global right ventricular (RV) hypokinesis; and generating an output comprising the heart disease risk score.

Proposed concepts thus aim to provide schemes, solutions, concepts, designs, methods and systems pertaining to predicting a probability of heart disease in a subject. In particular, embodiments aim to provide a method for predicting a probability of heart disease in a subject by providing an ECG signal of a subject to a trained machine-learning model which can predict and output a probability of the subject having at least one RWMA and/or global RV hypokinesis.

In other words, it is proposed that by providing a single ECG signal of a subject to a trained machine-learning model, a probability of the subject having at least one RWMA and/or a probability of the subject having global RV hypokinesis can be predicted. This therefore facilitates the predicting of whether a subject has either or both of at least one RWMA and/or global RV hypokinesis based on a commonly available ECG signal of the subject. This therefore provides a more efficient and/or more widely applicable method of predicting a probability of heart disease in a subject, and more specifically, wherein the heart disease comprises at least one of: at least one RWMA and global RV hypokinesis.

The inventors have realized that a machine-learning model, such as a CNN, can be trained and used to predict a probability of the subject having at least one RWMA and/or global RV hypokinesis based on an ECG signal rather than echocardiography. This therefore facilities the prediction of a subject having heart disease in a wider array of clinical circumstances, and also using a less expensive tool.

In a simple example, the present invention can be used to predict and output a probability of the subject having at least one regional wall motion abnormality. In another simple example, the present invention can be used to predict and output a probability of the subject having global RV hypokinesis. In another example, the present invention can be used to predict and output a probability of the subject having at least one regional wall motion abnormality and a separate probability of the subject having global RV hypokinesis. In a further example, the present invention may even be used to predict a location of a RWMA in a subject's heart.

It has been realized that a deep learning-based model can identify the presence of RWMA and/or global RV hypokinesis (and even potentially other heart diseases in addition, such as reduced left ventricular ejection fraction) based on analysis of ECG alone. Further, the model may even, in the presence of a high probability of RWMA, try to localize the abnormal regions of the myocardium. Identifying the presence of RWMAs is especially helpful for differentiating if a reduced left ventricular ejection fraction is from a focal abnormality or a global disease.

In some embodiments, the heart disease risk score may further comprise at least one of: a probability of the subject having more than one akinetic and/or dyskinetic wall; and a probability of the subject having a reduced left ventricular ejection fraction (LVEF). This therefore facilitates predicting the probability of the subject having other heart diseases in addition to RWMA and/or global RV hypokinesis.

In some embodiments, the trained machine-learning model may comprise a convolutional neural network (CNN). This has been found to be a particularly effective and/or efficient form of machine-learning model for predicting a heart disease risk score based on an ECG signal.

In some embodiments, the CNN may comprise a feature-extraction component and a classifier. This configuration of CNN has been found to be particularly effective and/or efficient for the present purpose.

In some embodiments, the feature-extraction component may comprise ResNet-50 architecture with convolutions specifically adapted for ECG signals. This has been found to make the CNN more efficient and/or effective for the present purpose.

In some embodiments, the feature-extraction component may comprise a convolutional layer which aggregates features. This has been found to make the CNN more efficient and/or effective for the present purpose.

In some embodiments, the classifier may comprise two fully connected layers with dropout before each layer. This has been found to make the CNN more efficient and/or effective for the present purpose.

In some embodiments, a sigmoid activation may be applied to each output of the classifier. This has been found to make the CNN more efficient and/or effective for the present purpose.

In some embodiments, the heart disease risk score may further comprise a location of a RWMA in the subject's heart. This can provide more clinically useful information.

In some embodiments, the location may comprise at least one of: anterior; anterolateral; anteroseptal; apical; inferior; inferolateral; and inferoseptal. These are all common locations for RWMA to be found in a subject's heart.

In some embodiments, the trained machine-learning model may be further trained to, if the predicted heart disease risk score is below a predetermined threshold, and based on the ECG signal, predict a heart disease future risk score indicating a probability of the subject having heart disease in the future; and wherein the generated output may comprise the heart disease future risk score. This can facilitate the outputting of clinically useful information even if the subject does not currently have heart disease (e.g., RWMA and/or global RV hypokinesis). Future can be understood as, for example, in the next five years.

In some embodiments, the ECG signal may comprise a 12-channel ECG waveform. This has been found to be a particularly beneficial form of ECG signal for predicting a probability of a subject having heart disease.

In some embodiments, the trained machine-learning model may be trained using a training algorithm configured to receive an array of training inputs and respective known outputs, wherein a training input comprises an ECG signal of a subject and a respective known output comprises a heart disease risk score indicating a probability of the subject having at least one RWMA and/or a probability of the subject having global RV hypokinesis. This has been found to be a beneficial form of training the machine-learning model.

In some embodiments, the ECG signal may have a ten second duration. This has been found to be a beneficial length for the ECG signal for compromising between efficiency and the amount of clinically useful information contained in the signal.

In some embodiments, the ECG signal may have a sampling frequency of 500Hz. This has been found to be a beneficial sampling frequency for the ECG signal for compromising between efficiency and the amount of clinically useful information contained in the signal.

In some embodiments, the method may further comprise obtaining the ECG signal of the subject. In some embodiments, obtaining the ECG signal of the subject may comprise capturing the ECG signal of the subject.

According to another aspect of the invention, there is provided a computer program comprising code means for implementing the method of any herein disclosed method when said program is run on a processing system.

According to another aspect of the invention, there is provided a processing system for predicting a probability of heart disease in a subject. The processing system comprising a processing unit configured to: provide an ECG signal of the subject as an input to a trained machine-learning model, the trained machine-learning model being trained to predict, based on the ECG signal, a heart disease risk score indicating a probability of the subject having at least one regional wall motion abnormality (RWMA) and/or a probability of the subject having global right ventricular (RV) hypokinesis; and generate an output comprising the heart disease risk score.

In some embodiments, the system may further comprise an input interface configured to obtain the ECG signal of the subject.

Thus, there may be proposed concepts for predicting a probability of heart disease in a subject, and this may be done based on providing an ECG signal of a subject to a machine-learning model trained to predict a heart disease risk score.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for predicting a probability of heart disease in a subject according to a proposed embodiment;
Fig. 2 is a flow diagram of a method for predicting a probability of heart disease in a subject according to a proposed embodiment;
Fig. 3 is a simplified block diagram of a system for predicting a probability of heart disease in a subject according to a proposed embodiment; and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to predicting a probability of heart disease in a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for predicting a probability of heart disease in a subject. Proposed concepts thus aim to provide schemes, solutions, concepts, designs, methods and systems pertaining to predicting a probability of heart disease in a subject. In particular, embodiments aim to provide a method for predicting a probability of heart disease in a subject by providing an ECG signal of a subject to a trained machine-learning model which can predict and output a probability of the subject having at least one RWMA and/or global RV hypokinesis respectively.

In other words, it is proposed that by providing a single ECG signal of a subject to a trained machine-learning model, a probability of the subject having at least one RWMA and/or global RV hypokinesis can be predicted. This therefore facilitates the predicting of whether a subject has either or both of at least one RWMA and/or global RV hypokinesis based on a commonly available ECG signal of the subject. This therefore provides a more efficient and/or more widely applicable method of predicting a probability of heart disease in a subject, and more specifically, wherein the heart disease comprises at least one of: at least one RWMA and global RV hypokinesis.

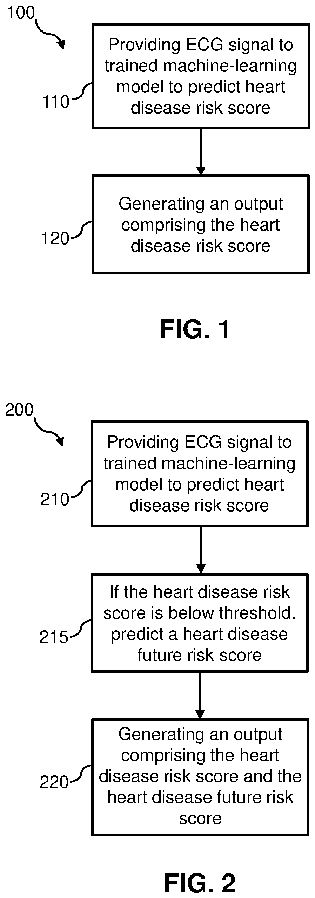

Referring now to Fig. 1, there is depicted a simplified flow diagram of a method 100 for predicting a probability of heart disease in a subject according to a proposed embodiment.

The method 100 begins with the step 110 of providing an ECG signal of the subject as an input to a trained machine-learning model, the trained machine-learning model being trained to predict, based on the ECG signal, a heart disease risk score indicating a probability of the subject having at least one regional wall motion abnormality (RWMA) and/or a probability of the subject having global right ventricular (RV) hypokinesis. In this embodiment, the heart disease risk score indicates a probability of the subject having at least one RWMA and a probability of the subject having global RV hypokinesis. In other embodiments, however, the heart disease risk score can indicate only one of these two options.

As a form of indication, the heart disease risk score can comprise at least one percentage probability, at least one fractional probability, at least one label (e.g., high-risk, medium-risk, low-risk), and/or at least one value between zero and one. In embodiments, such as this one, where the heart disease risk score indicates two probabilities, the heart disease risk score can comprise a single value, e.g., a single percentage, indicating a combined risk/probability of RWMA and global RV hypokinesis, or two separate values, e.g., two percentages, indicating a risk/probability of RWMA and global RV hypokinesis independently. For example, in this embodiment, the heart disease risk score comprises two percentage probabilities: a percentage probability of the subject having at least one RWMA, and a percentage probability of the subject having global RV hypokinesis.

It should be noted that hypokinesis, akinesis, and dyskinesis are different levels of severity for heart diseases affecting movement. In some embodiments, the trained machine-learning model can differentiate between hypokinetic, akinetic, and dyskinetic RWMA, i.e., can predict separate probabilities for each, or after predicting a probability for any form of RWMA, make a further prediction of whether it is hypokinetic, akinetic, or dyskinetic.

Step 120 comprises generating an output comprising the heart disease risk score. For example, this output can comprise a file comprising the heart disease risk score and/or it can comprise a display control signal describing the heart disease risk score. In some embodiments, the method can further comprise outputting the output to a display of an ECG monitor. In some embodiments, the method can further comprise outputting the output to an electronic health records management system. In some embodiments, the method can further comprise outputting the output to an ECG management system. In some embodiments, the method can further comprise, when the heart disease risk score exceeds a predetermined threshold, generating a notification or alarm.

In this embodiment, the trained machine-learning model is trained using a training algorithm configured to receive an array of training inputs and respective known outputs, wherein a training input comprises an ECG signal of a subject and a respective known output comprises a heart disease risk score indicating a probability of the subject having at least one RWMA and/or a probability of the subject having global RV hypokinesis. This has been found to be a beneficial form of training the machine-learning model. In other embodiments, however, the trained machine-learning model can be trained using any suitable training algorithm or in any suitable manner, as would be understood by the skilled person. For example, the training dataset (of training inputs and respective known outputs) can comprise real world data from around 20,000 patients. For example, the probabilities in the respective known outputs can comprise only 0% or 100%, i.e., whether the real-world patient either had the relevant heart disease or not. A portion of the training dataset (e.g., 20%) can then be used for testing the trained machine-learning model.

In an example, a trained machine-learning model was pre-trained using 235,568 ECG signals with weights randomly initialized using truncated normal initialization. An Adam optimizer was used with an initial learning rate of 3×10-4, ℓ2-weight decay of 1×10-4, default coefficients of β1=0.9 and β2=0.999, and a cosine annealing learning rate scheduler. To reduce model overfit, stochastic weighted averaging was used at each residual block and a dropout layer with P=0.5 for the fully connected layers. The model was trained using binary cross-entropy loss for multilabel classification with label smoothing of 0.05 for each output class. To reduce the rate of overfit and improve generalizability, a data augmentation can be applied by using random shifting, scaling, gaussian noise, and CutOut to the waveform. Fivefold cross-validation can then be used to assess robustness of the model during development. For example, models were trained for 100 epochs using an early stopping of 12 consecutive epochs with no improvement to the cross-validation macro AUC (area under the curve). The model with the highest validation AUC on the final cross-validation fold can then be selected as the trained machine-learning model.

In some embodiments, the method 100 can begin with the step (not shown) of obtaining the ECG signal of the subject. In some embodiments, obtaining the ECG signal of the subject can comprise capturing the ECG signal of the subject.

Referring now to Fig. 2, there is depicted a flow diagram of a method 200 for predicting a probability of heart disease in a subject according to a proposed embodiment.

Step 210 is much like step 110 of method 100 in that it comprises providing an ECG signal of the subject as an input to a trained machine-learning model, the trained machine-learning model being trained to predict, based on the ECG signal, a heart disease risk score indicating a probability of the subject having at least one regional wall motion abnormality (RWMA) and/or a probability of the subject having global right ventricular (RV) hypokinesis. In this embodiment, however, the heart disease risk score further comprises at least one of: a probability of the subject having more than one akinetic and/or dyskinetic wall; and a probability of the subject having a reduced left ventricular ejection fraction (LVEF). This therefore facilitates the probability of the subject having other heart diseases to be predicted in addition to RWMA and/or global RV hypokinesis.

For example, reduced LVEF can comprise LVEF ≤50%, LVEF ≤40%, and/or LVEF ≤35%.

In a particularly useful embodiment, the heart disease risk score comprises a probability of the subject having at least one RWMA and a probability of the subject having a reduced LVEF. Identifying the presence of RWMAs is especially helpful for differentiating if a reduced left ventricular ejection fraction is from a focal abnormality or a global disease.

In this embodiment, the trained machine-learning model comprises a convolutional neural network (CNN). This has been found to be a particularly effective and/or efficient form of machine-learning model for predicting a heart disease risk score based on an ECG signal. In other embodiments, however, the trained machine-learning model can take any suitable form, such as, for example, a random forest model or a recurrent neural network (RNN).

There are several types of neural network, such as, for example, convolutional neural networks (CNNs) and recurrent neural networks (RNNs). CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

In this embodiment, the CNN comprises a feature-extraction component and a classifier. This configuration of CNN has been found to be particularly effective and/or efficient for the present purpose, however, in other embodiments, any suitable form of CNN can be used, as would be understood by the skilled person.

In this embodiment, the feature-extraction component comprises ResNet-50 architecture with convolutions specifically adapted for ECG signals (i.e., lead-specific convolutions). The lead-specific convolutions allow the model to extract temporal features from each lead in parallel. This has been found to make the CNN more efficient and/or effective for the present purpose, however, in other embodiments, any suitable form of feature-extraction component can be used, as would be understood by the skilled person.

In this embodiment, the feature-extraction component comprises a convolutional layer which aggregates features. This layer can be the final layer of the feature-extraction component. This has been found to make the CNN more efficient and/or effective for the present purpose, however, in other embodiments, the feature-extraction component can take any suitable form, as would be understood by the skilled person. The aggregated (lead-specific) features, for example, can then be passed to a global average pooling layer for classification.

In this embodiment, the classifier comprises two fully connected layers with dropout before each layer. This has been found to make the CNN more efficient and/or effective for the present purpose, however, in other embodiments, the classifier can take any suitable form, as would be understood by the skilled person. This feature provides enhanced generalization by preventing overfitting. The dropout layers randomly deactivate a fraction of neurons during training, promoting robust feature learning and improving the model's ability to generalize well to unseen data. For example, the dropout layer can have P=0.5.

In this embodiment, a sigmoid activation is applied to each output of the classifier. This has been found to make the CNN more efficient and/or effective for the present purpose, however, this present invention can work without this feature, as would be understood by the skilled person. Applying a sigmoid activation to each output of the classifier ensures that the predicted values are scaled between 0 and 1, facilitating interpretation as probabilities.

Also, in this embodiment, the heart disease risk score further comprises a location of a RWMA in the subject's heart. This can provide more clinically useful information. Of course, the heart disease risk score would only comprise the location of the RWMA in the subject's heart if the subject has at least one RWMA. In some embodiments then, including this one, the trained machine-learning model only predicts a location of a RWMA in the subject's heart if the predicted probability of the subject having at least one RWMA is over a predetermined threshold, for example, 50%. In this embodiment, the location comprises at least one of: anterior; anterolateral; anteroseptal; apical; inferior; inferolateral; and inferoseptal. These are all common locations for RWMA to be found in a subject's heart (in accordance with the coronary perfusion territories), however, in other embodiments, the location can comprise any suitable location and/or method of location such as a set of coordinates, etc.

Localizing a RWMA is especially helpful in identifying patients with single vessel versus multivessel coronary artery disease, as well as complementing RWMA assessment during echocardiography, especially when views are suboptimal. Additionally, during repeat echocardiography for patients with suspected acute coronary syndrome this model may help monitor both the onset of new RWMA or changes in LVEF that require further evaluation and testing, for example.

In this embodiment, the ECG signal comprises a 12-channel ECG waveform. This has been found to be a particularly beneficial form of ECG signal for predicting a probability of a subject having heart disease, however, in other embodiments, any suitable form of ECG signal can be used, as would be understood by the skilled person. Further, in this embodiment, the ECG signal has a ten second duration and a sampling frequency of 500Hz. These have been found to be beneficial parameters for comprising between efficiency and the amount of clinically useful information contained in the signal. In other embodiments, however, the ECG signal can be of any suitable duration and have any suitable sampling frequency, as would be understood by the skilled person.

Step 215 comprises determining if the heart disease risk score is below a predetermined threshold. If the heart disease risk score comprises multiple probabilities, each probability can be independently checked against a predetermined threshold or the probabilities can be averaged and checked against a predetermined threshold. In some embodiments, each probability can have its own corresponding predetermined threshold to be compared against.

If the heart disease risk score is below the predetermined threshold, the trained machine-learning model predicts, based on the ECG signal, a heart disease future risk score indicating a probability of the subject having heart disease in the future. This can facilitate predicting and outputting clinically useful information even if the subject does not currently have heart disease (e.g., RWMA and/or global RV hypokinesis). Future can be understood as, for example, in the next five years. For example, the heart disease future risk score can indicate/comprise the probability of the subject having at least one RWMA in the next five years and/or the subject having global RV hypokinesis in the next five years. In different embodiments, the term 'future' can be understood as any appropriate time length, for instance, in the next one year, two years, three years, four years, five years, ten years, etc.

In this embodiment, the same trained machine-learning model is used to predict the heart disease risk score and the heart disease future risk score, but in other embodiments, different trained machine-learning models can be used.

In some embodiments, if the heart disease risk score is below the predetermined threshold (e.g., 50%) but above another predetermined threshold (e.g., 20%), then this value can be taken directly as the heart disease future risk score (perhaps modified in some way, e.g., multiplied by a predetermined factor) , i.e., without needing any machine-learning model to process the ECG signal again.

For example, it has been found that a method according to the present invention can identify patients at a threefold increased risk of RWMA, a threefold increased risk of acute coronary events, a fourfold increased risk of global RV hypokinesis, and a sixfold increased risk of LVEF ≤40% over the next five years. These findings suggest that a trained machine-learning model according to embodiments described herein can detect subtle or structural patterns in an ECG signal predictive of future ischemic events.

In some embodiments, the heart disease future risk score can further comprise an indication of a probability of the subject having at least one acute coronary event in the future (e.g., next five years). Example acute coronary events are myocardial ischemia or its acute complications, or a coronary revascularization procedure such as percutaneous angioplasty / stent placement or coronary artery bypass graft surgery.

In step 220, an output is generated comprising the heart disease risk score and the heart disease future risk score. In some embodiments, the output can comprise only the heart disease future risk score, i.e., the heart disease future risk score can be outputted instead of the heart disease risk score.

In summary, the inventors have developed a novel deep-learning based model to primarily identify and localize regional motion abnormalities using a (12-lead) ECG. In a real-world study, it was found that a trained machine-learning model such as the one used in method 200 was able to accurately detect abnormal wall motion in individual wall segments (AUC: 0.85 to 0.91), global RV hypokinesis (AUC: 0.88), LVEF ≤50% (AUC: 0.89) LVEF ≤40% (AUC: 0.92), and LVEF ≤35% (AUC: 0.93).

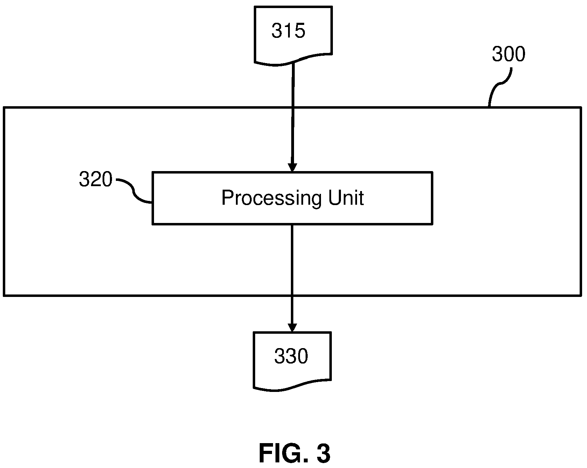

Referring now to Fig. 3, there is depicted a processing system 300 for predicting a probability of heart disease in a subject according to a proposed embodiment. The system 300 comprises a processing unit 320.

The system 300 is configured predict a probability of heart disease in a subject by processing an input 315. The input 315 comprises an ECG signal of the subject. The processing unit 320 is configured to provide the ECG signal 315 an input to a trained machine-learning model, the trained machine-learning model being trained to predict, based on the ECG signal, a heart disease risk score indicating a probability of the subject having at least one regional wall motion abnormality (RWMA) and/or a probability of the subject having global right ventricular (RV) hypokinesis. The processing unit 320 is further configured to generate an output 330. The output 330 comprises the heart disease risk score. In some embodiments, the processing unit 320 may comprise a CNN.

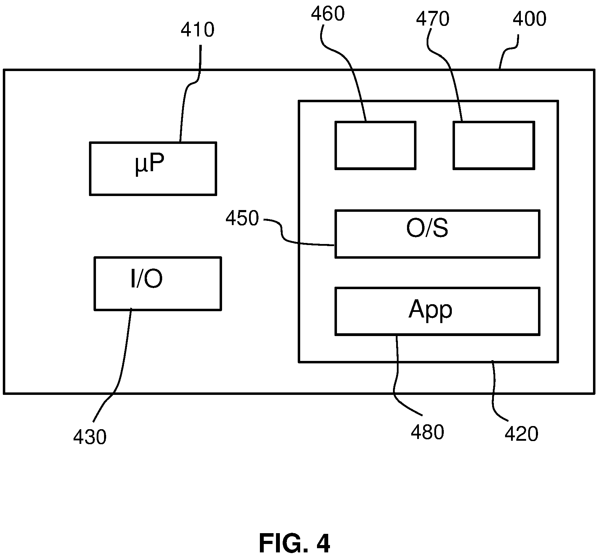

Fig. 4 illustrates an example of a computer 400 within which one or more parts of an embodiment may be employed. Various operations discussed above may utilize the capabilities of the computer 400. In this regard, it is to be understood that system functional blocks can run on a single computer or may be distributed over several computers and locations (e.g. connected via internet).

The computer 400 includes, but is not limited to, PCs, workstations, laptops, PDAs, palm devices, servers, storages, and the like. Generally, in terms of hardware architecture, the computer 400 may include one or more processors 410, memory 420 and one or more I/O devices 430 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 460, source code 470, and one or more applications 480 in accordance with exemplary embodiments. As illustrated, the application 480 comprises numerous functional components for implementing the features and operations of the exemplary embodiments. The application 480 of the computer 400 may represent various applications, computational units, logic, functional units, processes, operations, virtual entities, and/or modules in accordance with exemplary embodiments, but the application 480 is not meant to be a limitation.

The operating system 450 controls the execution of other computer programs, and provides scheduling, input-output control, file and data management, memory management, and communication control and related services. It is contemplated by the inventors that the application 480 for implementing exemplary embodiments may be applicable on all commercially available operating systems.

Application 480 may be a source program, executable program (object code), script, or any other entity comprising a set of instructions to be performed. When a source program, then the program is usually translated via a compiler (such as the compiler 460), assembler, interpreter, or the like, which may or may not be included within the memory 420, so as to operate properly in connection with the O/S 450. Furthermore, the application 480 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 430 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 430 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 430 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at start-up, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 400 pursuant to the software. The application 480 and the O/S 450 are read, in whole or in part, by the processor 410, perhaps buffered within the processor 410, and then executed.

When the application 480 is implemented in software it should be noted that the application 480 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 480 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-2, and the system of Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for predicting a probability of heart disease in a subject, the method comprising, the method comprising:
providing an ECG signal of the subject as an input to a trained machine-learning model (110), the trained machine-learning model being trained to predict, based on the ECG signal, a heart disease risk score indicating a probability of the subject having at least one regional wall motion abnormality, RWMA, and/or a probability of the subject having global right ventricular, RV, hypokinesis; and
generating an output (120) comprising the heart disease risk score.

2. The computer-implemented method of claim 1, wherein the heart disease risk score further comprises at least one of: a probability of the subject having more than one akinetic and/or dyskinetic wall; and a probability of the subject having a reduced left ventricular ejection fraction (LVEF).

3. The computer-implemented method of any of claims 1 or 2, wherein the trained machine-learning model comprises a convolutional neural network, CNN.

4. The computer-implemented method of claim 3, wherein the CNN comprises a feature-extraction component and a classifier.

5. The computer-implemented method of claim 4, wherein the feature-extraction component comprises ResNet-50 architecture with convolutions specifically adapted for ECG signals.

6. The computer-implemented method of claim 4 or 5, wherein the feature-extraction component comprises a convolutional layer which aggregates features.

7. The computer-implemented method of any of claims 3 to 6, wherein the classifier comprises two fully connected layers with dropout before each layer.

8. The computer-implemented method of any of claims 3 to 7, wherein a sigmoid activation is applied to each output of the classifier.

9. The computer-implemented method of any of claims 1 to 8, wherein the heart disease risk score further comprises a location of a RWMA in the subject's heart.

10. The computer-implemented method of claim 9, wherein the location comprises at least one of: anterior; anterolateral; anteroseptal; apical; inferior; inferolateral; and inferoseptal.

11. The computer-implemented method of any of claims 1 to 10, the trained machine-learning model is further trained to, if the predicted heart disease risk score is below a predetermined threshold, and based on the ECG signal, predict a heart disease future risk score (215) indicating a probability of the subject having heart disease in the future; and
wherein the generated output comprises the heart disease future risk score.

12. The computer-implemented method of any of claims 1 to 11 wherein the ECG signal comprises a 12-channel ECG waveform.

13. The computer-implemented method of any of claims 1 to 12, wherein the trained machine-learning model is trained using a training algorithm configured to receive an array of training inputs and respective known outputs, wherein a training input comprises an ECG signal of a subject and a respective known output comprises a heart disease risk score indicating a probability of the subject having at least one RWMA and/or a probability of the subject having global RV hypokinesis.

14. A computer program product comprising computer program code means which, when executed on a computing device having a processing system, cause the processing system to perform all of the steps of the computer-implemented method according to any of claims 1 to 13.

15. A processing system (300) for predicting a probability of heart disease in a subject, the processing system comprising:
a processing unit (320) configured to:
provide an ECG signal of the subject as an input to a trained machine-learning model, the trained machine-learning model being trained to predict, based on the ECG signal, a heart disease risk score indicating a probability of the subject having at least one regional wall motion abnormality, RWMA, and/or a probability of the subject having global right ventricular, RV, hypokinesis; and generate an output comprising the heart disease risk score.
